Europäisches Patentamt

European Patent Office

Office européen des brevets

(19)

(11) Veröffentlichungsnummer: **0 280 212 B1**

(12) **EUROPÄISCHE PATENTSCHRIFT**

(45) Veröffentlichungstag der Patentschrift: **29.05.91**

(51) Int. Cl.5: **C12N 11/08**

(21) Anmeldenummer: **88102459.0**

(22) Anmeldetag: **19.02.88**

Die Akte enthält technische Angaben, die nach dem Eingang der Anmeldung eingereicht wurden und die nicht in dieser Patentschrift enthalten sind.

(54) **Polyurethanmodifizierte Enzyme.**

(30) Priorität: **23.02.87 DE 3705687**

(43) Veröffentlichungstag der Anmeldung:
**31.08.88 Patentblatt 88/35**

(45) Bekanntmachung des Hinweises auf die
Patenterteilung:
**29.05.91 Patentblatt 91/22**

(84) Benannte Vertragsstaaten:
**AT BE CH DE ES FR GB GR IT LI LU NL SE**

(56) Entgegenhaltungen:
EP-A- 0 151 937     EP-A- 0 210 478
EP-A- 0 222 289     DE-A- 2 755 053
DE-A- 2 929 872     GB-A- 1 321 352

PATENT ABSTRACTS OF JAPAN, Band 10,
Nr. 103 (C-340)[2160], 18. April 1986; & JP-
A-60 232 089 (Toyo Gomu Kogyo K.K.)
18-11-1985

(73) Patentinhaber: **BOEHRINGER MANNHEIM GMBH**
**Patentabteilung, Abt. E Sandhofer Strasse**
**112-132 Postfach 31 01 20**
**W-6800 Mannheim 31 Waldhof(DE)**

(72) Erfinder: **Eicken, Ulrich, Dr. rer. nat.**
**Oppelner Weg 38 a**
**W-4000 Düsseldorf(DE)**
Erfinder: **Tischer, Wilhelm, Dr.**
**Finkenweg 5**
**W-8123 Peissenberg(DE)**

(74) Vertreter: **Huber, Bernhard, Dipl.-Chem. et al**
**Patentanwälte H. Weickmann, Dr. K. Fincke**
**F.A. Weickmann, B. Huber Dr. H. Liska, Dr. J.**
**Prechtel Möhlstrasse 22 Postfach 860 820**
**W-8000 München 86(DE)**

## Beschreibung

Die Erfindung betrifft polyurethanmodifizierte, wasserlösliche Enzyme sowie ein Verfahren zu ihrer Herstellung.

Es ist bekannt, Proteine wie z. B. Enzyme, zu modifizieren, um bestimmte Eigenschaften der Proteine, z. B. bestimmte physikalisch-chemische Eigenschaften, wie die Löslichkeit in einem gewünschten System (z. B. analytischem Reagenz), die Lagerstabilität, Verträglichkeit in einem hydrophoben Medium (z. B. bei der Bindung auf Kunststoffoberflächen) oder die biologischen Eigenschaften, wie die Erhöhung der Halbwertszeit eines Proteins im Plasma, die Verminderung der Antigenwirkung, die Aktivität in einem nicht wäßrigen System, insbesondere in einem organischen Lösungsmittel, zu verbessern.

Aus der DE-OS 27 55 053 ist es bekannt, biologisch wirksame Proteine durch Umsetzung mit einem Urethanpolymeren zu modifizieren. Danach wird ein biologisch wirksames Protein und ein in Wasser mindestens dispergierbares flüssiges Polyurethan-Vorpolymeres unter im wesentlichen wasserfreien Bedingungen zu einer Lösung umgesetzt, die dann in Wasser dispergiert wird. Ein Nachteil dieses Verfahrens ist es z. B., daß das biologisch aktive Protein als Trockenpulver eingesetzt werden muß, weil sonst wegen der Reaktivität der NCO-Gruppen mit Wasser ein Schaum oder ein hochvernetztes Produkt gebildet wird; weiters können nur Prepolymere verwendet werden, die bereits wasserlöslich sind. Durch die heterogene Reaktion eines wasserfreien Proteins mit den Prepolymeren wird außerdem in der Regel die Aktivität stark verringert oder sogar vollständig zerstört.

Aufgabe der vorliegenden Erfindung ist es, ein polyurethanmodifiziertes Enzym sowie Verfahren zu seiner Herstellung bereitzustellen, das die vorstehend genannten Nachteile vermeidet und mit dem ein modifiziertes Enzym bereitgestellt wird, das insbesondere die erwünschten Löslichkeitseigenschaften und biologischen Eigenschaften aufweist und mit dem eine wesentlich bessere Aktivitätsausbeute erhalten werden kann. Diese Aufgabe wird mit dem Gegenstand der vorliegenden Anmeldung gelöst.

Gegenstand der Erfindung ist ein polyurethanmodifiziertes, wasserlösliches Enzym, das erhältlich ist durch Umsetzung eines Enzyms in wäßriger Lösung mit einer wäßrigen Lösung eines Polyurethanprepolymer/Bisulfit-Addukts bei einem pH-Wert größer 7. Gegebenenfalls kann hierbei das Zusetzen eines organischen Cosolvens, das wassermischbar ist, z. B. Wasser/Dioxan, vorteilhaft sein.

Weiterer Gegenstand der Erfindung ist auch ein Verfahren zur Herstellung eines mit Polyurethan-modifizierten, wasserlöslichen Enzyms durch Umsetzung eines Enzyms mit einem wasserlöslichen Polyurethanprepolymer/Bisulfit-Addukt in wäßriger Lösung bei einem pH-Wert größer 7, das dadurch gekennzeichnet ist, daß man das Enzym in wäßriger Lösung mit einer wäßrigen Lösung des Polyurethanpräpolymer/Bisulfit-Addukts umsetzt.

Zweckmäßige Ausgestaltungen dieser Gegenstände gemäß den Ansprüchen 1 und 2 sind Gegenstand der Ansprüche 3 bis 9.

Überraschenderweise wurde gefunden, daß nach dem Verfahren der vorliegenden Erfindung polyurethanmodifizierte Enzyme erhalten werden können, die gegenüber den aus dem Stand der Technik bekannten polyurethanmodifizierten Enzymen eine wesentlich bessere Aktivitätsausbeute zeigen.

Vorzugsweise wird die Umsetzung so durchgeführt, daß man die wäßrige Lösung des Polyurethanprepolymer/Bisulfit-Addukts zu einer Lösung des Enzyms in einen wäßrigen Puffer mit einem pH-Wert größer 7 zugibt. Die Umsetzung wird vorzugsweise bei einem pH-Wert größer 8 und insbesondere bei einem pH-Wert größer 9 durchgeführt.

Alternativ kann bei der Umsetzung auch der umgekehrte Weg beschritten werden, nämlich die Lösung des Enzyms zu der Lösung des Polyurethanprepolymer/Bisulfit-Addukts zugegeben werden. Hierbei muß jedoch darauf geachtet werden, daß die Polymerlösung so gewählt ist, daß keine Hydrolyse stattfindet, z. B. wenn der pH < 7 ist.

Als Polyurethan-Komponente können NCO-terminierte Polyurethan-Prepolymere unterschiedlicher Funktionalität eingesetzt werden. Monofunktionelle, aus einem Monool und Diisocyanat erhaltene Polyurethane werden zur Enzymmodifikation vorzugsweise dann eingesetzt, wenn eine Vernetzung unerwünscht ist, z. B. für bekannte, therapeutisch verwendete Enzyme. Bifunktionelle, durch Umsetzung von Diol mit einem molaren Überschuß an Diisocyanat erhaltene Polyurethane besitzen den Vorteil einer großen Variabilität der Ausgangsmaterialien, wobei ein Aufbau nach dem Baukastenprinzip möglich ist (vgl. hierzu Encyclopedia of Polymer Science and Technology, S. 508 ff, Ullmann's Encyclopädie der techn. Chemie, Band 19, S. 303-310, Verlag Chemie 1983). Sie sind weiters leicht verarbeitbar, da keine oder kaum eine Vernetzung des Polymeren stattfindet. Bei der Enzymmodifikation mit diesen Polyurethanen können auch vernetzte modifizierte Enzyme erhalten werden. Polyfunktionelle Polyurethane, die durch Umsetzung von Polyol und Diisocyanat erhalten werden, können dann insbesondere vorteilhaft sein, wenn eine Vernetzung des modifizierten Enzyms ausdrücklich gewünscht wird.

Die verbleibenden reaktiven Isocyanatgruppen der Polyurethanprepolymeren werden dann mit Natriumbisulfit (NaHSO₃) in das erfindungsgemäß zur Modifizierung verwendete Polyurethanprepolymer/Bisulfit-Addukt überführt. Dies hat den Vorteil, daß durch die Einführung einer ionischen Gruppe die Hydrophilie der Polymeren wächst, so daß auch im nichtmodifizierten Zustand wasserunlösliche Polymere wasserlöslich gemacht werden können. Durch die Umsetzung wird die Reaktivität der NCO-Gruppen gegenüber Wasser verringert, wodurch es möglich wird, NCO-Gruppen vor einer Hydrolyse und Weiterreaktion zu schützen. Durch Auswahl der geeigneten Polyurethanprepolymere, durch die vernetzte Polymere, hydrophob-hydrophile Blockpolymere sowie eine Molekulargewichtsmodifizierung möglich ist, lassen sich die für die gewünschte Verwendung zweckmäßigsten Eigenschaften der polyurethanmodifizierten Enzyme erreichen.

Die Reaktionstemperatur und der pH-Wert der Umsetzung sollen so gewählt werden, daß keine Desaktivierung (Denaturierung) des Enzyms stattfindet; vorzugsweise wird bei Raumtemperatur gearbeitet, obwohl auch bei erhöhter Temperatur bis zur Denaturierungstemperatur des Enzyms oder unter Kühlung gearbeitet werden kann. Vorzugsweise wird bei einem pH-Wert von 7 bis 10 gearbeitet.

Das Verhältnis von Polyurethanprepolymer/Bisulfit-Addukt zum Enzym kann so gewählt sein, daß nach der Umsetzung noch freie, nicht umgesetzte NCO-Bisulfit-Addukt-Gruppen (Carbamoylsulfate) am Polymer vorhanden sind.

Ein allzu großer Überschuß an freien NCO-Bisulfit-Addukt-Gruppen sollte aber vermieden werden, weil sonst eine oft unerwünschte Kettenverlängerung eintreten könnte. Bei Vorhandensein eines Überschusses an freien NCO-Bisulfit-Addukt-Gruppen können diese durch Zugabe eines zusätzlichen NH₂-Gruppen-haltigen Reagenz (z. B. Lysin) jedoch abreagiert und dadurch eine unerwünschte Kettenverlängerung vermieden werden. Soll die Anzahl der an jedes Enzymmolekül gebundenen Polymerketten reduziert werden, was in bestimmten Fällen zweckmäßig sein kann, so wird mit einem Überschuß an Enzymaminogruppen gearbeitet. Das zweckmäßigste Verhältnis läßt sich, abhängig von den Eigenschaften des jeweiligen Enzyms (Löslichkeit, Anzahl der Aminogruppen pro Molekül etc.) und dem beabsichtigten Verwendungszweck, sowie den Eigenschaften des Polyurethanprepolymer/Bisulfit-Addukts (z. B. Anzahl der reaktiven Gruppen pro Gramm), leicht ermitteln, wobei ein Verhältnis der beiden Komponenten von 10:1 bis 1:10 bevorzugt ist.

Polyurethanprepolymere mit mindestens einer und vorzugsweise zwei endständigen Isocyanatgruppen, wie sie erfindungsgemäß eingesetzt werden, sind bekannt oder lassen sich nach an sich bekannten Verfahren synthetisieren. In Frage kommen z. B. alle für polyurethanmodifizierte Proteine aus dem Stand der Technik bekannten Polyurethanprepolymere (vgl. Ullmann, supra), die ein wasserlösliches Bisulfit-Addukt bilden und mit Wasser nicht weiter reagieren. Verwendbar sind z. B. die nicht-ionisch hydrophilierten Polyurethanprepolymeren aus Diisocyanaten mit Polyetherpolyolen, wie sie in der DE-OS 25 43 093 zur Herstellung wasserlöslicher Bisulfit-Additionsprodukte eingesetzt werden. Anstelle der Polyetherpolyole (vgl. Ullmann, supra) können aber auch Polyesterpolyole (vgl. Ullmann, supra) mit etwa dem gleichen Molekulargewicht eingesetzt werden; es ist auch möglich, die Polyetherpolyole oder Polyesterpolyole mit einem höherfunktionellen Polyisocyanat (vgl. Ullmann, supra z.B. Desmodur® N(Bayer)) umzusetzen. Bevorzugt sind aliphatische und cycloaliphatische Isocyanate, da ihre Bisulfit-Addukte stabiler als die der aromatischen Isocyanate sind.

Vorzugsweise werden als Polyurethanprepolymer-Komponente Polyurethanprepolymere eingesetzt, die ein Oxyalkylengerüst besitzen, vorzugsweise ein solches aus Oxyethylen-, oder auch insbesondere Oxypropylen- und/oder Oxybutylen-Einheiten, die sich von den entsprechenden Polyalkylenglykolen, z. B. Polyethylenglykol, vorzugsweise Polypropylenglykol und/oder insbesondere von Polybutylenglykol ableiten. Dabei kommen auch Block-Copolymere oder statistische Copolymere in Frage, insbesondere z. B. solche aus Ethylenoxid und Propylenoxid.

Das Polyurethanprepolymere kann auch ein Polyestergerüst, vorzugsweise ein lineares Polyestergerüst aufweisen, das sich insbesondere von einem Polyesterdiol ableitet, z. B. von einem Polyesterdiol aus einer zweiwertigen Säure, insbesondere mit 2 bis 6 Kohlenstoffatomen und einem Diol, insbesondere mit 2 bis 6 Kohlenstoffatomen und 2 bis 6 Sauerstoffatomen, z. B. Triethylenglykol (vgl. Ullmann, supra).

Erfindungsgemäß besonders bevorzugt sind Polyurethanprepolymere, die sich von Propylenglykol und insbesondere von Ethylenglykol ableiten oder Oxypropylen- und/oder Oxyethyleneinheiten zu mindestens 10, insbesondere 50 Mol-% enthalten.

Das Zahlenmittel des Molekulargewichts der Polyalkylenpolyole oder Polyesterpolyole liegt vorzugsweise zwischen 500 und 10000 Dalton, insbesondere zwischen 800 und 6000 Dalton und in erster Linie zwischen 800 und 2000 Dalton.

Bei Verwendung von Monoolen wird vorzugsweise ein solches verwendet, in dem die zweite OH-Gruppe der Polyalkylenglykole oder Polyesterdiole durch Veretherung oder Veresterung, z. B.

durch eine Methylgruppe, maskiert ist.

Als Diisocyanatkomponente für die Polyurethanprepolymeren kommen vorzugsweise aliphatische oder cycloaliphatische Diisocyanate in Frage, wie z. B. 1,4-Tetramethylen-diisocyanat, 1,10-Decamethylen-diisocyanat, Cyclohexylen-1,4-diisocyanat und insbesondere 1,6-Hexamethylen-diisocyanat; es kommen aber auch aromatische Diisocyanate, wie z. B. Toluol-2,6-diisocyanat oder andere üblicherweise zur Herstellung von Polyurethanen verwendete aromatische Diisocyanate in Frage.

Die Herstellung der erfindungsgemäß eingesetzten Polyurethanprepolymer/Bisulfit-Addukte kann auf an sich bekannte Weise erfolgen, z. B. wie in der DE-OS 25 43 093 angegeben.

Als Bisulfit-Komponente kommen salzartige, in Wasser lösliche Bisulfite in Frage; vorzugsweise werden Kalium- oder Ammonium-Bisulfit und insbesondere Natrium-Bisulfit, bzw. Natriumhydrogensulfit in Wasser gelöst, eingesetzt.

Die Umsetzung des Polyurethanprepolymers mit der Bisulfitverbindung zum Addukt kann in einem rein wäßrigen System, aber auch in einem Gemisch aus Wasser und einem mit Wasser mischbaren oder in Wasser löslichen organischen Lösungsmittel durchgeführt werden, wie z. B. in einem Gemisch aus Wasser/Dioxan; vorzugsweise werden dann eine Lösung des Polyurethanprepolymers in dem organischen Lösungsmittel und eine Lösung der Bisulfit-Verbindung in Wasser gemischt. Für die Zwecke der vorliegenden Erfindung ist est es bevorzugt, überschüssiges Diisocyanat (oder Polyisocyanat), niedermolekulare Polyadditionsprodukte niedermolekularer reaktiver NCO-Bisulfit-Addukte und/oder nicht umgesetzte Bisulfit-Verbindungen zu entfernen; diese Entfernung erfolgt vorzugsweise durch Dialyse.

Als Enzyme für die erfindungsgemäßen Polyurethan-modifizierten Enzyme kommen alle wasserlöslichen Enzyme in Frage, wie sie z. B. aus dem Stand der Technik bereits in modifizierter Form bekannt sind (vgl. z. B. DE-OS 27 55 053), also z. B. Oxido-Reduktasen, Lyasen, Transferasen, Isomerasen, Hydrolasen, Ligasen usw.

Die Reinheit des Enzyms ist dabei für die erfindungsgemäße Umsetzung von untergeordneter Bedeutung, d. h. es können reine Enzyme, unreine Enzyme, z. B. in Form von Extrakten usw. eingesetzt werden.

Die Konzentrationen von Polyurethanprepolymer/Bisulfit-Addukt und Enzym in den zur Umsetzung kommenden wäßrigen Lösungen sind nicht kritisch. Vorzugsweise beträgt die Konzentration des Polyurethanpräpolymer/Bisulfit-Addukts in der Lösung 0,1 bis 25 Gew.-%, insbesondere 4 bis 10 Gew.-%; die Konzentration des Enzyms 0,1 bis 5 und insbesondere 1 bis 2 Gew.-%. Diese Konzentrationen sind als Konzentrationen in den Lösungen vor dem Mischen zu verstehen.

Die nachfolgenden Beispiele sollen die Erfindung näher erläutern, ohne sie darauf zu beschränken. Wenn nicht anders angegeben, beziehen sich Teile- und Prozentangaben auf Gewichtsteile und Gewichtsprozente und Temperaturangaben auf die Celsius-Skala.

**Beispiel 1**

200 g wasserfreies Polyethylenglykol 2000 ($\triangleq$ 0,2 mol OH) (bezogen von der Firma Merck, Darmstadt) werden mit 23,5 g ($\triangleq$ 0,28 mol NCO) Hexamethylen-Diisocyanat unter Rühren bei 80° C umgesetzt, bis der NCO-Gehalt der Mischung 1,5 % beträgt ($\triangleq$ 0,28 mol NCO). Der NCO-Gehalt wird durch Umsetzung mit einem Überschuß von Di-n-butylamin nach Rücktitration mit Salzsäure gegen Bromphenolblau als Indikator bestimmt. Sobald der gewünschte Gehalt an NCO-Gruppen erreicht ist, werden 100 g Dioxan zugesetzt.

Von dem oben hergestellten Prepolymer werden 40,4 g, das entspricht 10 mM NCO-Gruppen, in 25 ml Dioxan gelöst. 1,05 g $Na_2S_2O_5$ oder eine äquivalente Menge $NaHSO_3$ werden in 25 ml Wasser gelöst und in einem Guß zu der Dioxanlösung zugegeben. Die Lösung wird dabei leicht trübe. Die Lösung wird noch 30 Minuten bei Raumtemperatur nachgerührt und dann in einen Dialyseschlauch eingefüllt und über Nacht gegen VE-Wasser dialysiert. Durch die Dialyse werden insbesondere nicht umgesetztes $Na_2S_2O_5$ und niedermolekulare Polyadditionsprodukte entfernt. Nach der Dialyse wird die Lösung einer Lyophilisation unterworfen. Das Polymer fällt als weißes Pulver an.

Der Gehalt der reaktiven Gruppen kann nach der Methode von G. B. Guise, J. Appl. Polymers Sci. 21 (1977) 3427 bestimmt werden. Die Werte liegen typischerweise im Bereich von 0,05 mM/g.

**Beispiel 2**

20 g wasserfreies Polypropylenglykol 1025 ($\triangleq$ 0,04 mol OH) (bezogen von der Firma Merck, Darmstadt) werden mit 5,4 g ($\triangleq$ 0,064 mol NCO) Hexamethylendiisocyanat unter Rühren bei 100° C so lange umgesetzt, bis ein NCO-Gehalt von 4 % erreicht ist.

Nach Zugabe von 5 g Dioxan wird die Lösung in einem Eisbad auf 4° C abgekühlt. Dann werden 100 ml kaltes Ethanol zugegeben und gerührt, bis eine homogene Lösung entstanden ist. Sofort danach werden 2,28 g $Na_2S_2O_5$ in 25 ml Wasser in einem Guß zugegeben. Die Mischung wird 30 Minuten bei 4° C und 30 Minuten bei Raumtemperatur nachgerührt.

Aus der Mischung werden das Ethanol und Dioxan am Rotationsverdampfer abgezogen und der Rückstand wird über Nacht gegen VE-Wasser dialysiert. Das erhaltene Produkt wird danach lyophilisiert. Das Polymer fällt als hochviskose, glasartige Flüssigkeit an.

## Beispiel 3

a) 46 mg alpha-Glucosidase (EC 3.2.1.20, aus Hefe, 50 U/mg gemessen bei 25°C mit Maltose als Substrat) werden in 3,1 ml 0,1 M Kaliumphosphat-Puffer (pH = 8,0) gelöst. 184 mg des im Beispiel 1 beschriebenen Prepolymers werden in 2 ml Wasser gelöst und zu der Enzymlösung zugegeben. Die Mischung wird 5 Stunden bei Raumtemperatur inkubiert. Danach wird die Mischung gegen 30 mM Kaliumphosphat (pH = 6,8) dialysiert.

Die Aktivitätsausbeute beträgt 60 %, bezogen auf die Anfangsaktivität.

b) Zum Vergleich wird alpha-Glucosidase wie oben behandelt, aber ohne Zusatz des Prepolymers.

c) In einem weiteren Vergleichsversuch wird die alpha-Glucosidase, wie oben beschrieben, mit einem Prepolymer inkubiert, dessen reaktive Gruppen mit Di-n-butylamin abgesättigt wurden.

Die nach 3 a), b) und c) erhaltenen drei Proben werden bei 37°C in 0,1 M Kaliumphosphat-Puffer (pH = 7,2) inkubiert. Dabei trüben die Enzyme aus den Vergleichsbeispielen 3 b) und 3 c) stark ein (die $E_{405}$ steigt von 0,205 auf 3,3), das nach 3 a) erfindungsgemäß modifizierte Enzym dagegen nur geringfügig (die $E_{405}$ steigt von 0,184 auf 0,281). Das Enzym ist somit trübungsfrei.

## Beispiel 4

30 mg Pen-G-Amidase (Penicillin-G-Amidase aus E. coli, E.C. 3.5.1.11, 10 U/mg, gemessen mit Penicillin G als Substrat bei 28°C) werden in 3 ml 0,1 M Triethanolamin-Puffer (pH = 8,5) gelöst. 120 mg des im Beispiel 1 beschriebenen Polymers werden in 3 ml desselben Puffers gelöst und zu der Enzymlösung zugegeben. Die Mischung wird 5 Stunden bei Raumtemperatur inkubiert.

Nach Beendigung der Umsetzung wird gegen 30 mM Phosphat (pH = 7,5) dialysiert und das erhaltene Produkt lyophilisiert.

Die Aktivitätsausbeute beträgt 57 % der Ausgangsaktivität.

Vergleichsbeispiel (Verfahren nach DE-OS 27 55 053).

1,5 g Polyethylenglykol 1500 (≙ 2 mmol OH) und 0,235 g Hexamethylendiisocyanat (HDI; ≙ 2,8 mmol NCO) werden gemischt und bei 80°C umgesetzt, bis der NCO-Gehalt 2 % beträgt.

Zu 1 g dieses Prepolymers werden dann 200 mg der im Beispiel 3 verwendeten alpha-Glucosidase (lyophilisiert) unter Rühren zugegeben und eine Stunde bei Raumtemperatur im Exsiccator belassen. Die Aufarbeitung erfolgt wie in der DE-OS 27 55 053 beschrieben, indem das Prepolymer-Enzym-Gemisch in Wasser gegebenenfalls unter Zusatz von Tensiden, z. B. Pluronic® gegeben wird. Nach Abtrennen der unlöslichen Anteile durch Filtration wird die enzymatische Aktivität gegen p-Nitrophenyl-alpha-D-Glucopyranosid gemessen.

Die gefundene Aktivität beträgt 2,2 % der eingesetzten Aktivität; im Vergleich dazu besitzt eine nach Beispiel 3 erhaltene erfindungsgemäße Polyurethanmodifizierte alpha-Glucosidase eine Aktivität von 60 %.

## Ansprüche

1. Mit Polyurethan modifiziertes, wasserlösliches Enzym, erhältlich durch Umsetzung eines Enzyms in wäßriger Lösung mit einer wäßrigen Lösung eines Polyurethanprepolymer/Bisulfit-Addukts bei einem pH-Wert größer 7.

2. Verfahren zur Herstellung eines mit Polyurethan modifizierten, wasserlöslichen Enzyms, **dadurch gekennzeichnet,** daß man das Enzym in wäßriger Lösung mit einer wäßrigen Lösung eines Polyurethanprepolymer/Bisulfit-Addukts bei einem pH größer 7 umsetzt.

3. Verfahren nach Anspruch 2, **dadurch gekennzeichnet,** daß man die wäßrige Lösung des Polyurethanprepolymer/Bisulfit-Adduktes zu einer Lösung des Enzyms in einem wäßrigen Puffer mit einem pH-Wert größer 7 zugibt.

4. Verfahren nach Anspruch 2 oder 3, **dadurch gekennzeichnet,** daß man die Umsetzung bei einem pH-Wert größer 8, insbesondere größer 9 durchführt.

5. Verfahren nach einem der Ansprüche 2 bis 4, **dadurch gekennzeichnet,** daß sich das Polyurethanprepolymere von einem Polyalkylenglykol oder einem Monoester oder Monoether davon ableitet.

**6.** Verfahren nach Anspruch 5, **dadurch gekennzeichnet,** daß das Polyalkylenglykol ein Polyethylenglykol, Polypropylenglykol, ein Block-Copolymeres oder ein statistisches Copolymeres von Ethylenglykol und Propylenglykol ist.

**7.** Verfahren nach einem der Ansprüche 2 bis 4, **dadurch gekennzeichnet,** daß sich das Polyurethanprepolymere von einem Polyesterdiol oder einem Monoester oder Monoether davon ableitet.

**8.** Verfahren nach einem der Ansprüche 2 bis 7, **dadurch gekennzeichnet,** daß das Zahlenmittel des Molekulargewichts des Polyurethanprepolymers zwischen 500 und 10000 Dalton, insbesondere zwischen 800 bis 2000 Dalton liegt.

**9.** Mit Polyurethan modifiziertes, wasserlösliches Enzym nach Anspruch 1, **dadurch gekennzeichnet,** daß man die Umsetzung des Enzyms in wäßriger Lösung mit einer wäßrigen Lösung eines Polyurethanprepolymer/Bisulfit-Adduktes gemäß einem der Ansprüche 3 bis 8 durchführt.

Patentansprüche für folgende Vertragsstaaten : ES, GR

**1.** Verfahren zur Herstellung eines mit Polyurethan modifizierten, wasserlöslichen Enzyms, **dadurch gekennzeichnet,** daß man das Enzym in wäßriger Lösung mit einer wäßrigen Lösung eines Polyurethanprepolymer/Bisulfit-Addukts bei einem pH-Wert größer 7 umsetzt.

**2.** Verfahren nach Anspruch 1, **dadurch gekennzeichnet,** daß man die wäßrige Lösung des Polyurethanprepolymer/Bisulfit-Addukts zu einer Lösung des Enzyms in einem wäßrigen Puffer mit einem pH-Wert größer 7 zugibt.

**3.** Verfahren nach Anspruch 1 oder 2, **dadurch gekennzeichnet,** daß man die Umsetzung bei einem pH-Wert größer 8, insbesondere größer 9 durchführt.

**4.** Verfahren nach einem der Ansprüche 1 bis 3, **dadurch gekennzeichnet,** daß sich das Polyurethanprepolymere von einem Polyalkylenglykol oder einem Monoester oder Monoether davon ableitet.

**5.** Verfahren nach Anspruch 4, **dadurch gekennzeichnet,**

daß das Polyalkylenglykol ein Polyehylenglykol, Polypropylenglykol, ein Block-Copolymeres oder ein statistisches Copolymeres von Ethylenglykol und Propylenglykol ist.

**6.** Verfahren nach einem der Ansprüche 1 bis 3, **dadurch gekennzeichnet,** daß sich das Polyurethanprepolymere von einem Polyesterdiol oder einem Monoester oder Monoether davon ableitet.

**7.** Verfahren nach einem der Ansprüche 1 bis 6, **dadurch gekennzeichnet,** daß das Zahlenmittel des Molekulargewichts des Polyurethanprepolymers zwischen 500 und 10000 Dalton, insbesondere zwischen 800 bis 2000 Dalton liegt.

**Claims**

1. Water-soluble enzyme modified with polyurethane, obtainable by reaction of an enzyme in aqueous solution with an aqueous solution of a polyurethane pre-polymer/bisulphite adduct at a pH value greater than 7.

2. Process for the preparation of a water-soluble enzyme modified with polyurethane, characterised in that one reacts the enzyme in aqueous solution with an aqueous solution of the polyurethane pre-polymer/ bisulphite adduct at a pH value greater than 7.

3. Process according to claim 2, characterised in that one adds the aqueous solution of the polyurethane pre-polymer/bisulphite adduct to a solution of the enzyme in an aqueous buffer with a pH value greater than 7.

4. Process according to claim 2 or 3, characterised in that one carries out the reaction at a pH value greater than 8, especially greater than 9.

5. Process according to one of claims 2 to 4, characterised in that the polyurethane pre-polymer is derived from a polyalkylene glycol or a monoester or monoether thereof.

6. Process according to claim 5, characterised in that the polyalkylene glycol is a polyethylene glycol, polypropylene glycol, a block co-polymer or a statistical co-polymer of ethylene glycol and propylene glycol.

7. Process according to one of claims 2 to 4, characterised in that the polyurethane pre-polymer is derived from a polyester diol or a monoester or monoether thereof.

8. Process according to one of claims 2 to 7, characterised in that the numerical average of the molecular weight of the polyurethane pre-polymer lies between 500 and 10000 Dalton, especially between 800 and 2000 Dalton.

9. Water-soluble enzyme modified with polyurethane according to claim 1, characterised in that one carries out the reaction of the enzyme in aqueous solution with an aqueous solution of a polyurethane pre-polymer/bisulphite adduct according to one of claims 3 to 8.

Claims for the following Contracting States : GR, ES

1. Process for the preparation of a water-soluble enzyme modified with polyurethane, characterised in that one reacts the enzyme in aqueous solution with an aqueous solution of a polyurethane pre-polymer/ bisulphite adduct at a pH value greater than 7.

2. Process according to claim 1, characterised in that one adds the aqueous solution of the polyurethane pre-polymer/bisulphite adduct to a solution of the enzyme in an aqueous buffer with a pH value greater than 7.

3. Process according to claim 1 or 2, characterised in that one carries out the reaction at a pH value greater than 8, especially greater than 9.

4. Process according to one of claims 1 to 3, characterised in that the polyurethane pre-polymer is derived from a polyalkylene glycol or a monoester or monoether thereof.

5. Process according to claim 4, characterised in that the polyalkylene glycol is a polyethylene glycol, polypropylene glycol, a block co-polymer or a statistical co-polymer of ethylene glycol and propylene glycol.

6. Process according to one of claims 1 to 3, characterised in that the polyurethane pre-polymer is derived from a polyester diol or a monoester or monoether thereof.

7. Process according to one of claims 1 to 6, characterised in that the numerical average of the molecular weight of the polyurethane pre-polymer lies between 500 and 10000 Dalton, especially between 800 and 2000 Dalton.

**Revendications**

1. Enzyme hydrosoluble, modifiée par du polyuréthane, qui peut être obtenue par réaction d'une enzyme en solution aqueuse avec une solution aqueuse d'un composé d'addition prépolymère de polyuréthane/bisulfite à une valeur de pH supérieure à 7.

2. Procédé pour la préparation d'une enzyme hydrosoluble, modifiée par du polyuréthane, caractérisé en ce qu'on fait réagir l'enzyme en solution aqueuse avec une solution aqueuse d'un composé d'addition prépolymère de polyuréthane/bisulfite à une valeur de pH supérieure à 7.

3. Procédé selon la revendication 2, caractérisé en ce qu'on ajoute la solution aqueuse du composé d'addition prépolymère de polyuréthane/bisulfite à une solution de l'enzyme dans un tampon aqueux ayant une valeur de pH supérieure à 7.

4. Procédé selon la revendication 2 ou 3, caractérisé en ce qu'on effectue la réaction à une valeur de pH supérieure à 8, en particulier supérieure à 9.

5. procédé selon l'une des revendications 2 à 4, caractérisé en ce que le prépolymère de polyuréthane dérive d'un polyalkylèneglycol ou d'un monoester ou monoéther de celui-ci.

6. Procédé selon la revendication 5, caractérisé en ce que le polyalkylèneglycol est un polyéthylèneglycol, polypropylèneglycol, un copolymère bloc ou un copolymère statistique de l'éthylèneglycol et du propylèneglycol.

7. Procédé selon l'une des revendications 2 à 4, caractérisé en ce que le prépolymère de polyuréthane dérive d'un polyester-diol ou d'un monoester ou monoéther de celui-ci.

8. Procédé selon l'une des revendications 2 à 7, caractérisé en ce que la moyenne numérique du poids moléculaire du prépolymère de polyuréthane se situe entre 500 et 10000 daltons, en particulier entre 800 jusqu'à 2000 daltons.

9. Enzyme hydrosoluble, modifiée par du polyuréthane selon la revendication 1, caractérisée en ce qu'on effectue la réaction de l'enzyme en solution aqueuse avec une solution aqueuse d'un produit d'addition prépolymère de polyuréthane/bisulfite selon l'une des revendications 3 à 8.

Revendications pour les Etats contractants suivants : GR, ES

1. Procédé de préparation d'une enzyme hydrosoluble, modifiée par du polyuréthane, caractérisé en ce qu'on fait réagir l'enzyme en solution aqueuse avec une solution aqueuse d'un produit d'addition prépolymère de polyuréthane/bisulfite à une valeur de pH supérieure à 7.

2. Procédé selon la revendication 1, caractérisé en ce qu'on ajoute la solution aqueuse du produit d'addition prépolymère de polyuréthane/bisulfite à une solution de l'enzyme dans un tampon aqueux ayant une valeur de pH supérieure à 7.

3. Procédé selon la revendication 1 ou 2, caractérisé en ce qu'on effectue la réaction à un valeur de pH supérieure à 8, en particulier supérieure à 9.

4. Procédé selon l'une des revendications 1 à 3, caractérisé en ce que le prépolymère de polyuréthane dérive d'un polyalkylèneglycol ou d'un monoester ou moncéther de celui-ci.

5. Procédé selon la revendication 4, caractérisé en ce que le polyalkylèneglycol est un polyéthylèneglycol, polypropylèneglycol, un copolymère bloc ou un copolymère statistique de l'éthylèneglycol et du propylèneglycol.

6. Procédé selon l'une des revendications 1 à 3, caractérisé en ce que le prépolymère de polyuréthane dérive d'un polyester-diol ou d'un monoester ou monoéther de celui-ci.

7. Procédé selon l'une des revendications 1 à 6, caractérisé en ce que la moyenne numérique du poids moléculaire du prépolymère de polyuréthane se situe entre 500 et 10000 daltons, en particulier entre 800 jusqu'à 2000 daltons.